# EUROPEAN PATENT SPECIFICATION

(11) **EP 2 229 208 B1**
(45) Date of publication and mention of the grant of the patent: **22.05.2013**
(21) Application number: 08860198.4
(22) Date of filing: 10.12.2008
(51) Int. Cl.: A61M 21/02, A61B 5/0484

(54) **PROCEDURE FOR OPTIMISING THE PARAMETERS OF THE LIGHT PULSES EMITTED BY LEDS APPLIED TO A PAIR OF GLASSES.**
VERFAHREN ZUR OPTIMIERUNG DER PARAMETER DER LICHTPULSE, DIE VON LEDS AUF EINER BRILLE AUSGESENDET WERDEN
PROCÉDURE D'OPTIMISATION DES PARAMÈTRES D'IMPULSIONS LUMINEUSES ÉMISES PAR DES DELS ET APPLIQUÉES À UNE PAIRE DE LUNETTES

(30) Priority: 11.12.2007 IT VI20070313
(43) Date of publication of application: 22.09.2010
(73) Proprietor: Ferro Milone, Francesco, 36100 Vicenza (IT)
(72) Inventor: Ferro Milone, Francesco, 36100 Vicenza (IT)
(74) Representative: Bonini, Ercole
(86) International application number: PCT/EP2008/067261
(87) International publication number: WO 2009/074624

(56) References cited:
- EP-A- 0 412 629
- WO-A-00/47157
- WO-A-03/011104
- WO-A-2008/147958
- FR-A- 2 554 605
- US-A- 5 242 376
- US-A- 5 709 645

## Description

The invention concerns a procedure for optimising the parameters of the light pulses emitted by leds applied to a pair of glasses.

The invention substantially concerns a procedure for programming the microprocessor that controls the switching on and off of leds applied to a pair of glasses.

It has been known for some time now that our brain sends out electromagnetic waves, which can be recorded through the electroencephalogram, whose frequency depends on the activity of the brain itself.

For example, during its normal activity our brain sends out waves that are conventionally classified as beta (β) and gamma (γ) waves whose frequency varies from 13 to 80 Hertz, depending on the individual and on the activity performed.

If the individual is in a condition of mental relaxation, the frequency of the waves sent out by the brain varies from 8 to 13 Hertz and the waves included in this range are called alpha waves (α).

Furthermore, if the individual is half asleep or is sleeping, the brain sends out waves whose frequency is even lower, in the order of 0.3-7 Hertz, and which are classified as delta (δ) and theta (θ) waves.

In the range of the brain waves whose frequency is included between 0.3 and 80 Hertz and also through electroencephalograms it has been observed that each individual has his/her own specific peak of spectral density for each one of the groups of the frequencies mentioned above.

It is also known that the stimulating effect obtained, for example, through leds positioned in front of the closed eyes with the typical frequency of α or β-γ or δ-θ waves, if repeated over time has the effect of increasing the amplitude of the frequency peak emitted by the brain.

Substantially, therefore, it is possible to say that there is a direct interaction between the light stimulation and the response of the waves sent out by the brain, which means that said waves are amplified compared to those normally emitted.

Since the α, β-γ and δ-θ waves correspond to different types of brain activity, it has also been possible to establish that α waves are related, more than others, to the individual's ability to memorise events.

More specifically, it has been observed that the ability to memorise is directly proportional to the amplitude of the α waves emitted by the individual and also to their frequency.

For example, the frequency of α waves, which varies from 8 to 13 Hertz, in younger individuals with greater memorisation ability tends to reach 13 Hertz, while in adults or the elderly it can drop to 8 Hertz or be even lower.

Since light stimulation has proved to have positive effects, different devices have been developed which, with different purposes and different application forms, interfere with the brain's activity.

For example, Patent US 5 709 645 concerns a device consisting of a pair of glasses in which the leds are switched on and off according to a predefined frequency. This device is used to allow the person wearing such a pair of glasses to relax completely.

In order to improve the relationship between the waves emitted by the brain of a given individual and the light stimulation produced by the led, according to the European Patent EP 0 412 629 a device has been developed that is substantially constituted by an helmet to be worn by the user, wherein said helmet, by means of EEG (Electroencephalogram) devices, records the specific frequency of the α waves sent out by the user's brain so that, on the base of said recording, the light stimulation is amplified and repeated through the leds with the same frequency recorded by the EEG (electroencephalogram).

In Patent EP 0 412 629, the signals recorded by the electroencephalogram are substantially used in a continuous way so that they interact with the light pulse emission circuit and that a light pulse that is switched on and off with the same frequency recorded by the EEG is continuously transmitted.

As far as the applicant knows, the frequency supplied to the leds in the various known devices is either fixed or maintained constantly equal to that of the α waves of the user's brain, as in the case just described.

Experimental data have shown that the response of the data supplied by the electroencephalogram varies according to different parameters that are not only those related to the frequency that is specific to the waves emitted by the brain.

The response of the EEG is influenced by the frequency of the light pulses but also by the colour emitted by the leds, the light intensity of the same, the duration of the duty cycle of each single light pulse, as well as by the time of application of said light pulses to the individual.

In practice, each individual responds to the light stimulation in an optimal way, that is, amplifying the response of the frequency typical of the α waves, or even shifting the frequency towards higher values, according to a plurality of parameters that are applied to the leds.

The object of the present invention is to design and implement a procedure that makes it possible to program a microprocessor (CPU) to be applied to the frame of a pair of glasses with the parameters relating to the colour emitted by the leds, the light intensity, the frequency and the duty cycle duration, in such a way as to obtain in response the maximum amplitude of the waves emitted by the brain, thus achieving also a substantial stability of the results.

A further object of the invention is to develop a procedure that makes it possible to program a microprocessor for glasses suitable for a specific user, comprising only a first step for setting the microprocessor through analysis of the results given by the EEG devices, in order to successively free the user from any other obligation and finally supply him/her with a pair of glasses that are already programmed for his/her specific needs in order to maximise the results that can be achieved.

These and other objects will be highlighted in greater detail in the description of the procedure that is the subject of the present invention, according to the contents of the first claim.

Other details and advantages of the invention are highlighted in greater detail in the description of a preferred embodiment of the invention supplied as an example without limitation, with reference to the attached drawings, wherein:
- Figure 1 shows a view of the pair of glasses comprising the CPU and the plurality of leds;
- Figure 2 shows a view of the cable connection between the pair of glasses and the processor;
- Figure 3 shows a view of the connection between the processor and the EEG device;
- Figure 4 illustrates step a) of the procedure that is the subject of the invention;
- Figure 5 illustrates step i) of the procedure that is the subject of the invention;
- Figure 6 is a block diagram of the elements that make up the pair of glasses and of their connections.

The procedure that is the subject of the invention is particularly and advantageously suited to be applied to a pair of glasses comprising a plurality of leds, a microprocessor (CPU) and a battery suited to ensure power supply to the plurality of leds and the CPU.

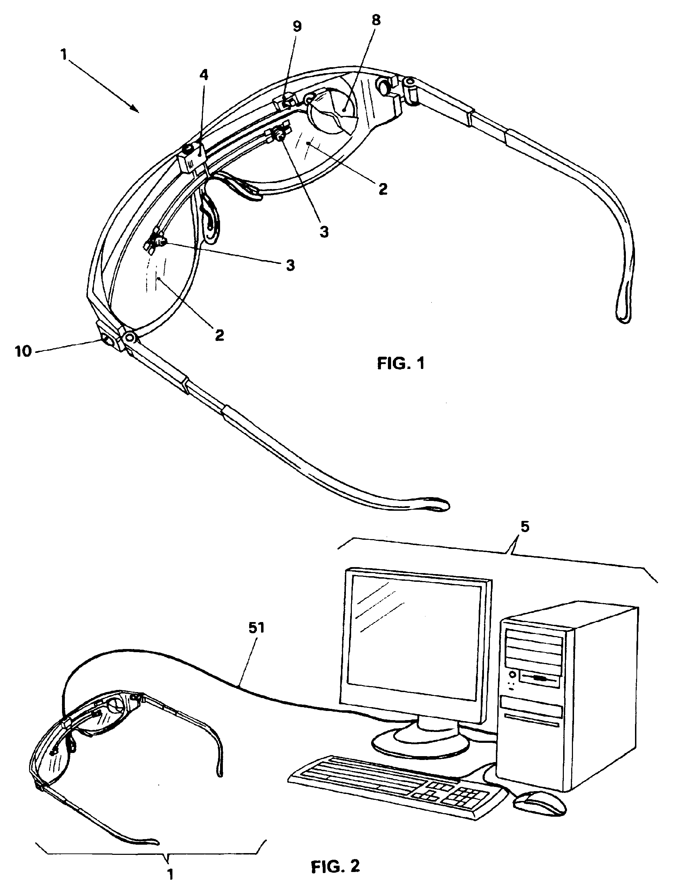

In particular, as shown in Figure 1, the pair of glasses **1** has its front area **2** darkened and, when the glasses are worn by a person, the plurality of leds **3** is centered at the level of the eyeballs.

The plurality of leds **3** comprises a group of leds that sends out red light pulses, a second group that emits green light pulses, a third group that emits blue light pulses, while finally switching on the three groups at the same time makes it possible to obtain white light pulses.

The switching on and off of each led is controlled by a CPU **4** present on the pair of glasses **1.**

As regards the CPU **4** present on the glasses **1,** it is of the programmable type and it can be connected to a processor **5** via the connection cable **51** in order to transmit data in both directions, as can be observed in Figure 2.

In other construction variants, the connection of the CPU **4** located on the pair of glasses **1** with the processor **5** can take place through the wireless Bluetooth or a similar technology.

It is important to underline that, during the implementation of the procedure of the invention, the connection between the glasses **1** and the processor **5** isn't continuous, but is activated only during the steps in which data transmission between the two pieces of equipment is required.

On the contrary, during the remaining steps the pair of glasses **1** is an independent and insulated device.

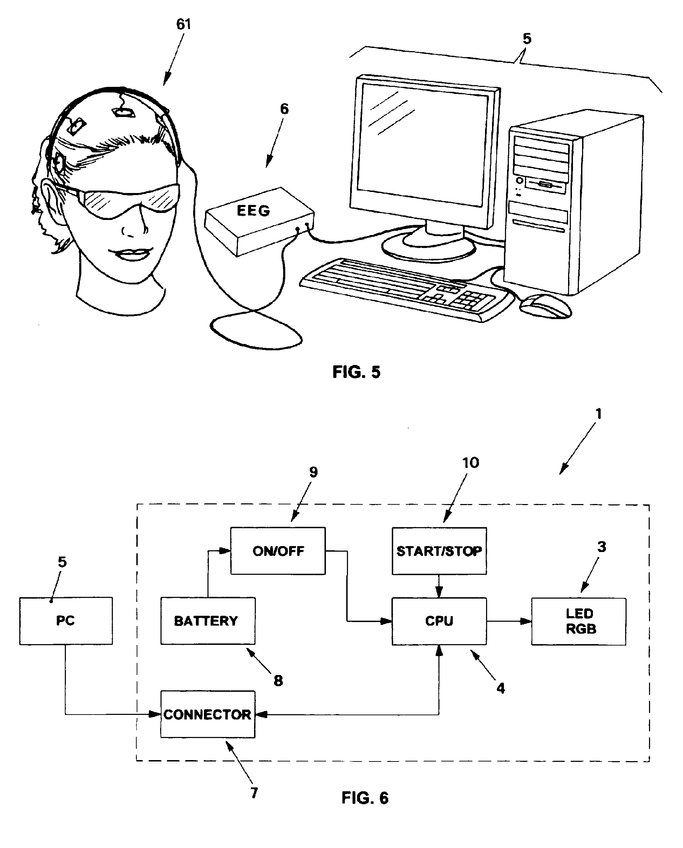

In particular, as far as the specific composition of the pair of glasses **1** is concerned, it can be observed in Figure 6 that the CPU **4** can be connected to the processor **5** through the interposition of connection means **7** and, furthermore, between the battery **8** and the CPU **4** there is a switch **9** that makes it possible to open or close the power supply flow to the CPU.

Finally, the CPU **4** is electrically connected to a switch **10** that allows the emission of light pulses by the pair of glasses **1** to be activated and stopped.

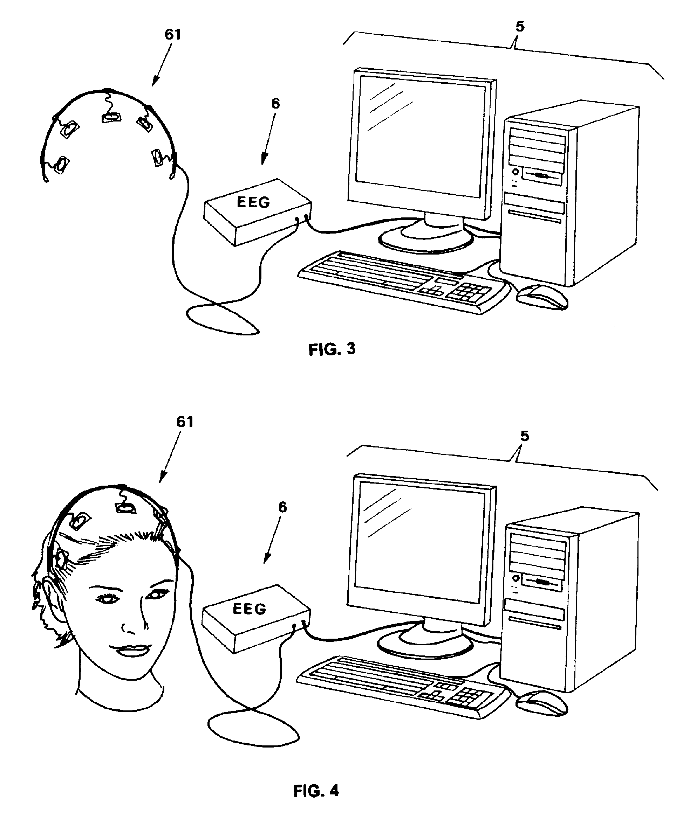

As to the processor **5,** as shown in Figure 3 it is connected, in its turn, to a device **6** for carrying out the EEG test and it is provided with a software **S1** that is particularly suitable for reading, storing and displaying the data received by and/or transmitted to the pair of glasses and the EEG device.

In its turn, the EEG device **6** is connected to the pair of glasses **1** so that, during the EEG test, the presence or absence of the light pulses emitted by the glasses **1** is signalled by the device itself.

The optimisation procedure that is the subject of the invention, as shown in Figure 4, includes a first step indicated by a), during which the operator positions a plurality of electrodes **61,** belonging to the EEG device **6** and configured according to the international 10-20 system, on the head of the person being examined, in order to successively carry out the EEG test.

This examination is performed without the aid of the pair of glasses mentioned above, in such a way as to allow the characteristics of the brain waves of the person under examination to be recorded without external limitations.

The most significant cerebral waves are those called α, β-γ and δ-θ.

In further construction variants, more than one EEG examinations can be performed on the person being examined and successively the results obtained can be processed with statistical methods, in such a way as to obtain a single, more reliable result.

After recording the diagram of the whole spectral density resulting from the EEG, the second step of the procedure, indicated by b), consists in analysing the final result obtained and determining the maximum peak of the spectral density for each frequency interval included between 0.3 and 80 Hertz.

A further step of the procedure, indicated by c), includes first of all selecting one of the frequency intervals mentioned above, that is α, β-γ or δ-θ and successively setting, in the software **S1** present in the processor **5,** the frequency of the maximum peak identified within the range of the selected frequency.

In particular, the operator can identify and set the frequency value with a minimum resolution of 0.1 Hertz.

According to the invention, the successive step d) includes setting, in the apposite sections of the software **S1** located in the processor **5,** the predefined values of the parameters that determine the characteristics of the light pulse emission by the plurality of leds.

These parameters are respectively the colour, intensity and duration of each single light pulse emitted by the plurality of leds **3** of the pair of glasses **1.**

In particular, as far as the colour of the light pulses is concerned, this can be selected, as already mentioned, among the primary colours, that is, red, green, blue and finally white.

The light intensity of each single pulse is included within a range of possible values from one millicandela up to 80 millicandela.

Finally, the duration of a single pulse may vary from one and 99 per cent of the time period that elapses between the beginning of a pulse and the beginning of the successive pulse.

Furthermore, according to the successive step e), a further parameter is set in the software **S1** located in the processor **5,** said parameter corresponding to the duration of the entire emission cycle of the light pulses at the selected frequency.

Once all the parameters have been set in the processor **5,** according to step f) said parameters are transferred to the CPU **4** located in the pair of glasses **1,** so that the light pulses are emitted with the established characteristics, in one or more cycles, as will be explained in greater detail below.

This, as already mentioned, can be done using the cable **51** that connects the computer **5** to the CPU **4** applied to the pair of glasses **1,** or via Blutooth or similar technology.

The successive step g) consists in having the person to be examined wear the pair of glasses **1** with the CPU **4** just programmed and capable of controlling the light pulses of the plurality of leds **3** according to the parameters set during steps d) and e).

In this way, the light pulse emission cycle is started, supplying power to the plurality of leds **3** present on the pair of glasses.

After operating, in step f), the plurality of leds **3** according to the set parameters and treatment time, it is possible to proceed to step i) that, as shown in Figure 5, consists in the repetition of the EEG examination with the equipment **6,** with the difference, compared to the preceding example, that the examination is performed simultaneously to the emission of the light pulses by the plurality of leds **3,** in such a way as to evaluate the effectiveness of said emission for the individual undergoing the treatment.

As explained above, it is possible to carry out several EEG examinations in the same operating conditions, finally processing the results with statistical methods in order to obtain a single result.

At the end of this operation, it is possible to proceed to step I), which includes reading and analysing the obtained result, wherein a comparison is made between the amplitude and frequency values of the maximum peak recorded within the frequency interval previously selected among the α, β-γ and δ-θ intervals and the maximum peak within the same frequency interval identified during the first EEG examination, without the aid of the pair of glasses **1.**

Said data are successively stored together with the colour, intensity and duration parameters in order to set the light pulse emission characteristics.

In order to allow the effect of the emission of the light pulses on an individual and his/her reaction to be evaluated, step m) includes repeating the EEG examination with the aid of the glasses **1,** as shown in Figure 5, each time modifying a single parameter selected among colour, intensity and duration but substantially maintaining the same frequency selected at the beginning.

The repetition cycle of the EEG examinations ends after verification of the most significant, if not all the possible combinations of the three parameters mentioned above, always maintaining the same pulse frequency selected at the beginning.

According to the procedure that is the subject of the invention, after the EEG examinations there is step n), which consists in identifying, among all the data collected, the maximum response peak obtained within the selected interval and, consequently, in selecting the colour, intensity and duration parameters of the single pulse that have allowed this maximum response peak to be obtained.

The last operation to be performed corresponds to step o), consisting in the retransmission of the parameters that allow the best result to be achieved from the processor **5** to the CPU **4** present on the glasses **1,** through the apposite software **S1** provided in the processor **5** and via cable connections **51** or Blutooth or similar technology.

Sometimes it may be necessary to carry out several consecutive light pulse emission cycles with different setting parameters.

In practice, it is possible to program the CPU **4** provided in the pair of glasses **1** with several consecutive pulse emission cycles through one or more repetitions of the procedure of the invention, each time selecting a different frequency interval among α, β-γ or δ-θ and setting, as frequency of the light pulses, the frequency of the maximum spectral density peak within the selected interval.

Upon implementation, the procedure that is the subject of the invention may be subjected to further changes or variations that, even if not described herein and not illustrated in the drawings, must all be considered protected by the present patent, provided that they fall within the scope of the following claims. Where the technical characteristics illustrated in the claims are followed by reference numbers, these are provided for the sole purpose of facilitating the reader and said reference numbers shall consequently have no restrictive effect on the coverage of each element identified as an example.

## Claims

1. Procedure for optimising the adjustment parameters of the light pulses emitted by a plurality of leds (3) applied to a pair of glasses (1), said pair of glasses (1) comprising a CPU (4) and at least one battery (8) for supplying power to said CPU (4) and said plurality of leds (3), said pair of glasses (1) being connected to a processor (5) that in turn is connected to a device (6) for carrying out the EEG examination, said processor (5) being equipped with a software (S1) for reading, storing and displaying the collected and/or transmitted data,
**characterised in that** it comprises the following steps:
a) performing said at least one EEG examination on an individual without using said pair of glasses (1);
b) analysing the results of said at least one EEG examination displayed in said processor (5) and identifying the maximum peak of each frequency interval α, β-γ and δ-θ within the entire spectral density recorded by said at least one EEG examination;
c) selecting one of the frequency intervals between 0.3 and 80 Hertz (α, β-γ and δ-θ) and setting, in said software (S1) provided in said processor (5), the frequency of the maximum peak relating to said selected interval;
d) selecting and setting, in said software (S1) provided in said processor (5), a predefined value for each one of the parameters, that is, colour, intensity and duration (duty cycle) of each single light pulse;
e) selecting and setting, in said software (S1) provided in said processor (5), the duration of the emission of the light pulses;
f) transferring said parameters mentioned in points d) and e) to said CPU (4) provided in said pair of glasses (1);
g) having a person wear said pair of glasses (1);
h) switching on said plurality of leds (3) according to the settings described in point f);
i) submitting said person to said at least one EEG examination during the emission of the light pulses according to the settings described in points d) and e);
l) reading and storing the results of said at least one EEG examination carried out during step i) and comparing the values of amplitude and frequency of the maximum peak recorded within the frequency interval selected during step c) with the peak of the same frequency interval identified during step b);
m) repeating the steps in sequence from d) to I), modifying only one parameter at a time, choosing among colour, intensity and duration (duty cycle) of each single light pulse, and registering the data collected with said at least one EEG;
n) identifying, among all the data collected with the EEG, the maximum reaction peak obtained at the peak frequency identified during step b) within the frequency interval selected during step c) and identifying the values of the colour, intensity and duration (duty cycle) parameters of each single pulse that have made it possible to obtain the above mentioned maximum reaction peak;
o) transferring the frequency, colour, intensity and duration (duty cycle) parameters of the single pulse identified during step n) to the CPU (4) provided in said pair of glasses (1).

2. Procedure according to claim 1), **characterised in that** it includes the following steps:
- selecting at least one frequency interval between α, β-γ and δ-θ (between 0.3 and 80 Hertz), different from the one selected during step c) as mentioned in claim 1);
- setting, in said software (S1) provided in said processor (5), the frequency of the maximum peak relating to said selected interval;
- repeating the steps from d) to o) at the set frequency.

3. Procedure according to any of the preceding claims, **characterised in that** the frequency interval α comprises the frequencies from 8 to 13 Hertz.

4. Procedure according to any of the preceding claims, **characterised in that** the frequency interval β-γ comprises the frequencies from 13 to 80 Hertz.

5. Procedure according to any of the preceding claims, **characterised in that** the frequency interval δ-θ comprises the frequencies from 0.3 to 7 Hertz.

6. Procedure according to any of the preceding claims, **characterised in that** the colour of each single light pulse is selected among red, green, blue and white.

7. Procedure according to any of the preceding claims, **characterised in that** the intensity of each single light pulse varies between 1 and 80 millicandela.

8. Procedure according to any of the preceding claims, **characterised in that** the duration (duty cycle) of each single light pulse varies between 1 and 99 percent of the period of time that elapses between the beginning of one pulse and the beginning of the successive pulse.

9. Procedure according to any of the preceding claims, **characterised in that** the frequency value of the maximum peak identified and set during step c) as described in claim 1) has a minimum resolution of 0.1 Hertz.

10. Procedure according to any of the preceding claims, **characterised in that** said pair of glasses (1) is connected to said processor (5) via a cable (51).

11. Procedure according to any of the claims from 1) to 9), **characterised in that** said pair of glasses (1) is connected to said processor (5) via a Bluetooth connection.

## Patentansprüche

1. Verfahren zur Optimierung der Einstellparameter für die von einer Vielzahl an einer Brille (1) angebrachter Leds (3) abgegebenen Lichtimpulse, wobei diese Brille (1) eine CPU (4) und wenigstens eine Batterie (8) zur Versorgung der besagten CPU (4) und der besagten Vielzahl von Leds (3) mit Strom umfasst, wobei die besagte Brille (1) mit einem Prozessor (5) verbunden ist, der seinerseits an eine Vorrichtung (6) zur Ausführung der EEG-Untersuchung angeschlossen ist, wobei der besagte Prozessor (5) mit einer Software (S1) zum Lesen, Speichern und Anzeigen der erfassten und/oder übermittelten Daten versehen ist,
**dadurch gekennzeichnet, dass** es die folgenden Schritte umfasst:
a) Ausführung der basagten, wenigstens einen EEG-Untersuchung an einer Person ohne Verwendung der besagten Brille (1);
b) Analyse der in dem besagten Prozessor (5) angezeigten Ergebnisse der besagten, wenigstens einen EEG-Untersuchung und Identifikation des maximalen Spitzenwerts jedes Frequenzintervalls α, β-γ und δ-θ über die gesamte, durch die besagte wenigstens eine EEG-Untersuchung aufgezeichnete Spektraldichte;
c) Auswahl von einem der Frequenzintervalle zwischen 0,3 und 80 Hertz (α, β-γ und δ-θ) und Einstellung der Frequenz des maximalen Spitzenwerts für das besagte, ausgewählte Intervall in der besagten, im besagten Prozessor (5) residenten Software (S1);
d) Auswahl und Einstellung eines vorbestimmten Werts für jeden der Parameter, das heißt Farbe, Stärke und Dauer (Arbeitszyklus) jedes einzelnen Lichtimpulses in der besagten, im besagten Prozessor (5) residenten Software (S1);
e) Auswahl und Einstellung der Dauer der Abgabe der Lichtimpulse in der besagten, im besagten Prozessor (5) residenten Software (S1);
f) Übertragung der besagten Parameter aus den Punkten d) und e) an die besagte, an der besagten Brille (1) befindliche CPU (4);
g) Aufsetzen der besagten Brille (1) an einer Person;
h) Einschaltung der besagten Vielzahl von Leds (3) gemäß den in Punkt f) beschriebenen Einstellungen;
i) Ausführung der wenigstens einen EEG-Untersuchung an einer Person während der Abgabe der Lichtimpulse gemäß den in den Punkten d) und e) beschriebenen Einstellungen;
l) Lesung und Speicherung der Ergebnisse der in Schritt i) ausgeführten, besagten wenigstens einen EEG-Untersuchung und Vergleich der Amplituden- und Frequenzwerte des innerhalb des in Schritt c) gewählten Frequenzintervalls aufgezeichneten, maximalen Spitzenwerts mit dem Spitzenwert des gleichen, während Schritt b) identifizierten Frequenzintervalls;
m) Wiederholung der Schritte von d) bis I), wobei jeweils nur ein Parameter auf einmal geändert wird, und zwar wahlweise die Farbe, Stärke und Dauer (Arbeitszyklus) jedes einzelnen Lichtimpulses sowie Aufzeichnung der mit dem besagten, wenigstens einen EEG erfassten Werte;
n) Identifikation, aus allen mit dem EEG erfassten Daten, des maximalen Reaktions-Spitzenwerts, der bei der während Schritt b) identifizierten Spitzenfrequenz innerhalb des während Schritt c) gewählten Frequenzintervalls erzielt wurde, und Identifikation der Werte der Farb-, Stärken- und Dauerparameter (Arbeitszyklus) jedes einzelnen Impulses, welche die Erzielung des oben genannten maximalen Reaktions-Spitzenwerts ermöglicht haben;
o) Übertragung der während der Phase n) identifizierten Parameter Frequenz, Farbe, Stärke und Dauer (Arbeitszyklus) des einzelnen Impulses an die auf der besagten Brille (1) befindliche CPU (4).

2. Verfahren gemäß Patentanspruch 1), **dadurch gekennzeichnet, dass** es folgende Schritte umfasst:
- Auswahl von wenigstens einem anderen Frequenzintervall zwischen α, β-γ und δ-θ (zwischen 0,3 und 80 Hertz) als jenem, das in Schritt c) aus Patentanspruch 1) gewählt worden war;
- Einstellung der Frequenz des Spitzenwerts des besagten, gewählten Intervalls in der besagten, im besagten Prozessor (5) residenten Software (S1);
- Wiederholung der Schritte von d) bis o) auf der eingestellten Frequenz.

3. Verfahren gemäß eines jeden der vorstehenden Patentansprüche, **dadurch gekennzeichnet, dass** das Frequenzintervall α die Frequenzen von 8 bis 13 Hertz umfasst.

4. Verfahren gemäß eines jeden der vorstehenden Patentansprüche, **dadurch gekennzeichnet, dass** das Frequenzintervall β-γ die Frequenzen von 13 bis 80 Hertz umfasst.

5. Verfahren gemäß eines jeden der vorstehenden Patentansprüche, **dadurch gekennzeichnet, dass** das Frequenzintervall δ-θ die Frequenzen von 0,3 bis 7 Hertz umfasst.

6. Verfahren gemäß eines jeden der vorstehenden Patentansprüche, **dadurch gekennzeichnet, dass** die Farbe jedes einzelnen Lichtimpulses aus Rot, Grün, Blau und Weiß gewählt wird.

7. Verfahren gemäß eines jeden der vorstehenden Patentansprüche, **dadurch gekennzeichnet, dass** die Stärke jedes einzelnen Lichtimpulses zwischen 1 und 80 Milli-Candela variiert.

8. Verfahren gemäß eines jeden der vorstehenden Patentansprüche, **dadurch gekennzeichnet, dass** die Dauer (Arbeitszyklus) jedes einzelnen Lichtimpulses zwischen 1 und 99 Prozent der zwischen dem Beginn eines Impulses und dem Beginn des nachfolgenden Impulses verstreichenden Zeitperiode variiert.

9. Verfahren gemäß eines jeden der vorstehenden Patentansprüche, **dadurch gekennzeichnet, dass** der Frequenzwert des im unter Patentanspruch 1) beschriebenen Schritt c) identifizierten und eingestellten Spitzenwerts eine Mindestauflösung von 0,1 Hertz hat.

10. Verfahren gemäß eines jeden der vorstehenden Patentansprüche, **dadurch gekennzeichnet, dass** die besagte Brille (1) über ein Kabel (51) mit dem besagten Prozessor (5) verbunden ist.

11. Verfahren gemäß eines jeden der Patentansprüche von 1) bis 9), **dadurch gekennzeichnet, dass** die besagte Brille (1) über eine Bluetooth-Verbindung mit dem besagten Prozessor (5) verbunden ist.

## Revendications

1. Procédé d'optimisation des paramètres de réglage des impulsions lumineuses émises par une pluralité de DEL (3) appliquée à une paire de lunettes (1), ladite paire de lunettes (1) comprenant une CPU (4) et au moins une batterie (8) d'alimentation de ladite CPU (4) et de ladite pluralité de DEL (3), ladite paire de lunettes (1) étant reliée à un élaborateur (5) qui a son tour est relié à un dispositif (6) pour l'exécution de l'examen EEG, ledit élaborateur (5) étant doté d'un logiciel ad hoc (S1) pour la lecture, la mise en mémoire et l'affichage des données recueillies et/ou transmises :
**caractérisé en ce qu**'il comprend les phases suivantes:
a) exécuter ledit au moins un examen EEG sur une personne sans l'utilisation de ladite paire de lunettes (1);
b) analyser les résultats dudit au moins un examen EEG, affichés dans ledit élaborateur (5), et identifier le pic maximum de chaque intervalle de fréquence α, β-γ et δ-θ, dans l'entière densité spectrale détectée par ledit au moins un examen EEG;
c) sélectionner un desdits intervalles de fréquence entre 0,3 et 80 Hertz
(α, β-γ e δ-θ) et introduire, dans ledit logiciel (S1) se trouvant dans ledit élaborateur (5), la fréquence du pic maximum relatif audit intervalle choisi;
d) sélectionner et introduire, dans ledit logiciel (S1) se trouvant dans ledit élaborateur (5), une valeur prédéfinie de chacun des paramètres de couleur, intensité et durée (cycle de travail) de chaque impulsion lumineuse unique;
e) sélectionner et introduire, dans ledit logiciel (S1) se trouvant dans ledit élaborateur (5), le temps de durée de l'émission des impulsions lumineuses;
f) transférer lesdits paramètres indiqués dans les points d) et e) à ladite CPU (4) présente sur ladite paire de lunettes (1);
g) faire porter ladite paire de lunettes (1) à une personne;
h) commander l'allumage de ladite pluralité de DEL (3) selon les introductions mentionnées au point f);
i) exécuter ledit au moins un examen EEG sur ladite personne durant l'émission des impulsions lumineuses selon les introductions mentionnées aux points d) et e);
l) lire et mettre en mémoire les résultats dudit au moins un examen EEG exécuté durant la phase i) et comparer les valeurs de la largeur et de la fréquence du pic maximum détecté à l'intérieur de l'intervalle de fréquence sélectionné durant la phase c) avec le pic du même intervalle de fréquence identifié durant la phase b);
m) répéter les phases en séquence de d) à I) en modifiant chaque fois un seul paramètre choisi entre couleur, intensité et durée (cycle de travail) de chaque impulsion lumineuse unique, et enregistrer les données recueillies avec ledit au moins un EEG;
n) identifier, parmi toutes les données recueillies par l'EEG, le pic maximum de réaction obtenu à la fréquence de pic identifiée durant la phase b) à l'intérieur de l'intervalle de fréquence sélectionné durant la phase c) et détecter les valeurs de paramètres de couleur, intensité et durée (cycle de travail) de chaque impulsion qui a permis d'obtenir ledit pic maximum de réaction mentionné ci-dessus;
o) transférer les paramètres de fréquence, couleur, intensité et durée (cycle de travail) de l'impulsion unique identifiés durant la phase n) à la CPU (4) se trouvant sur ladite paire de lunettes (1).

2. Procédé selon la revendication 1), **caractérisé en ce q**u'il comprend les phases suivantes:
- sélectionner au moins un intervalle de fréquence, entre α, β-γ et δ-θ (entre 0,3 et 80 Hertz), différent de celui sélectionné durant la phase c), comme indiqué dans la revendication 1);
- introduire, dans ledit logiciel (S1) se trouvant dans ledit élaborateur (5), la fréquence du pic maximum relatif audit intervalle sélectionné;
- répéter les phases de d) à o) à la fréquence introduite.

3. Procédé selon l'une quelconque des revendications précédentes, **caractérisé en ce que** l'intervalle de fréquence α comprend les fréquences de 8 à 13 Hertz.

4. Procédé selon l'une quelconque des revendications précédentes, **caractérisé en ce que** l'intervalle de fréquence β-γ comprend les fréquences de 13 à 80 Hertz.

5. Procédé selon l'une quelconque des revendications précédentes, **caractérisé en ce que** l'intervalle de fréquence δ-θ comprend les fréquences de 0,3 à 7 Hertz.

6. Procédé selon l'une quelconque des revendications précédentes, **caractérisé en ce que** la couleur de chaque impulsion lumineuse unique est sélectionnée parmi les couleurs rouge, vert, bleu et blanc.

7. Procédé selon l'une quelconque des revendications précédentes, **caractérisé en ce que** l'intensité de chaque impulsion lumineuse unique varie entre 1 et 80 millibougies.

8. Procédé selon l'une quelconque des revendications précédentes, **caractérisé en ce que** la durée (cycle de travail) de chaque impulsion lumineuse unique varie entre 1 et 99 pourcent de la période de temps qui s'écoule entre le début d'une impulsion et le début de l'impulsion successive.

9. Procédé selon l'une quelconque des revendications précédentes, **caractérisé en ce que** la valeur de fréquence du pic maximum détecté et introduit durant la phase c), comme indiqué dans la revendication 1), présente une résolution minimum de 0,1 Hertz.

10. Procédé selon l'une quelconque des revendications précédentes, **caractérisé en ce que** ladite paire de lunettes (1) est reliée audit élaborateur (5) au moyen d'un câble (51).

11. Procédé selon l'une quelconque des revendications de 1) à 9), **caractérisé en ce que** ladite paire de lunettes (1) est reliée audit élaborateur (5) au moyen d'une connexion Bluetooth.
